# EUROPÄISCHE PATENTANMELDUNG

(11) **EP 1 504 786 A1**
(43) Veröffentlichungstag der Anmeldung: **09.02.2005**
(21) Anmeldenummer: 04008564.9
(22) Anmeldetag: 08.04.2004
(51) Int. Cl.: A61M 25/10

(54) **Katheteranordnung**

(30) Priorität: 08.08.2003 EP 03017591
(71) Anmelder: Acrostak Corp., 8409 Winterthur (CH)
(72) Erfinder: Schwager, Michael, 8404 Winterthur (CH)
(74) Vertreter: Weiss, Wolfgang

(57) **Zusammenfassung**

Die Erfindung betrifft eine Katheteranordnung (1) mit einem Innenschaft (2) zur Aufnahme eines Führungsdrahtes (3) und eines expandierbaren Ballons (4), wobei der Ballon (4) am proximalen und/oder distalen Ende (5, 6) einen schrägen Bereich (7, 8) aufweist.

## Beschreibung

Die Erfindung betrifft eine Katheteranordnung mit einem Innenschaft zur Aufnahme eines Führungsdrahtes und eines expandierbaren Ballons.

Derartige Katheteranordnungen dienen dazu, expandierbare Ballons an Verengungsstellen in Körpergefässen, beispielsweise Arterien, zu befördern, um Stents zu implantieren, Medikamente an die kranken Stellen zu bringen, Verengungen aufzuweiten etc.

Zur Führung des Katheters wird in bekannter Weise ein steuerbarer Führungsdraht verwendet, wobei sich bei der Einführung des Katheters in das Gefäss speziell bei Gefässabzweigungen Probleme ergeben. Der Führungsdraht wird hierbei weit vorgeschoben, um eine zufrieden stellende Führung im Bereich der Gefässabzweigung zu gewährleisten. Aus anatomischen Gründen ist häufig eine solche Einführung des Katheters nicht möglich.

Der vorzuschiebende Katheter ist wesentlich steifer als die Führungsdrahtspitze. Der Führungsdraht kann keine genügend grosse Biegekraft auf den Katheter ausüben und wird bei den bekannten Katheteranordnungen somit häufig aus der Gefässabzweigung herausgedrückt, wenn der steifere Katheter entlang des Führungsdrahtes in Richtung auf die Gefässverzweigung vorgeschoben wird.

In der DE 100 25 266 A1 ist eine solche Katheteranordnung beschrieben. Hierbei weist der Innenschaft eine über das distale Ende des Ballons hinausragende gebogene Spitze auf.

Wie bei dem vorgenannten Stand der Technik ist es somit Aufgabe der Erfindung eine Katheteranordnung vorzuschlagen, die einerseits eine sichere Einführung des Katheters mit dem geeignet gefalteten Ballon gewährleistet. Ein Herausdrücken oder unerwünschtes Hemmen des Führungsdrahtes bei der Einführung in das Gefäss soll vermieden werden.

Erfindungsgemäss wird die Aufgabe dadurch gelöst, dass der Ballon am proximalen und/oder distalen Ende einen schrägen Bereich aufweist. Bei der erfindungsgemässen Katheteranordnung sind an den Ballonenden ein- und/oder beidseitig Schrägen angeordnet. Diese Schrägen dienen dazu, eine kontrollierbare Steifigkeit einzustellen, die im wesentlichen von dem Winkel und damit der Länge der Schrägen abhängig ist. In vorteilhafter Weise beträgt die Länge des schrägen Bereichs 0,5 bis 50 mm, vorzugsweise 2 bis 6 mm. Gemäss besonderer Ausführungsformen kann eine erfindungsgemässe Schräge am proximalen und/oder distalen Ende des Ballons angeordnet sein, wobei der Innenschaft mit dem Ballon verschweisst ist.

Gemäss einer weiteren bevorzugten Ausführungsform weist die Katheteranordnung eine Schutzhülle auf, die über den Ballon vorzugsweise durch Drehung aufgebracht wird. Um den Ballon geeignet falten zukönnen, wird die Schutzhülle über den Innenschaft über den Ballon durch eine Drehbewegung aufgebracht. Um die Katheteranordnung mit der erfindungsgemässen Schräge optimal einsetzbar zu machen, weist die Schutzhülle ebenfalls an einem und/oder beiden Enden jeweils eine Schräge auf. Diese Schräge dient dazu, den Ballon mit der Katheteranordnung geeignet zu falten. Der Winkel der Schräge beträgt vorteilhafterweise 30° bis 45°. Vorteilhaft kann ein Ende der Schutzhülle das Ballonende überragen, welches nach dem Aufbringen der Schutzhülle in Richtung Ballon abgeknickt wird. Diese Massnahme dient der Sicherheit bei der Anwendung. Diese Abknickung ist so gestaltet, dass sie gut sichtbar ist. Es wird damit vermieden dass der Ballon irrtümlich mit der Schutzhülle in das Blutgefäss eingeführt wird.

Weitere Einzelheiten ergeben sich aus den Unteransprüchen und der folgenden Figurenbeschreibung von Ausführungsbeispielen.

### Es zeigen

Fig. 1 eine vereinfachte Schnittdarstellung der erfindungsgemässen Katheteranordnung am distalen Ende,
Fig. 2 eine vereinfachte Schnittdarstellung der erfindungsgemässen Katheteranordnung am proximalen Ende,
Fig. 3 eine erfindungsgemässe Katheteranordnung im Bereich einer Abzweigung und
Fig. 4 eine Schutzhülle über den gefalteten Ballon
Fig. 5 eine Ausführungsform mit abgeknicktem Schutzhüllenende

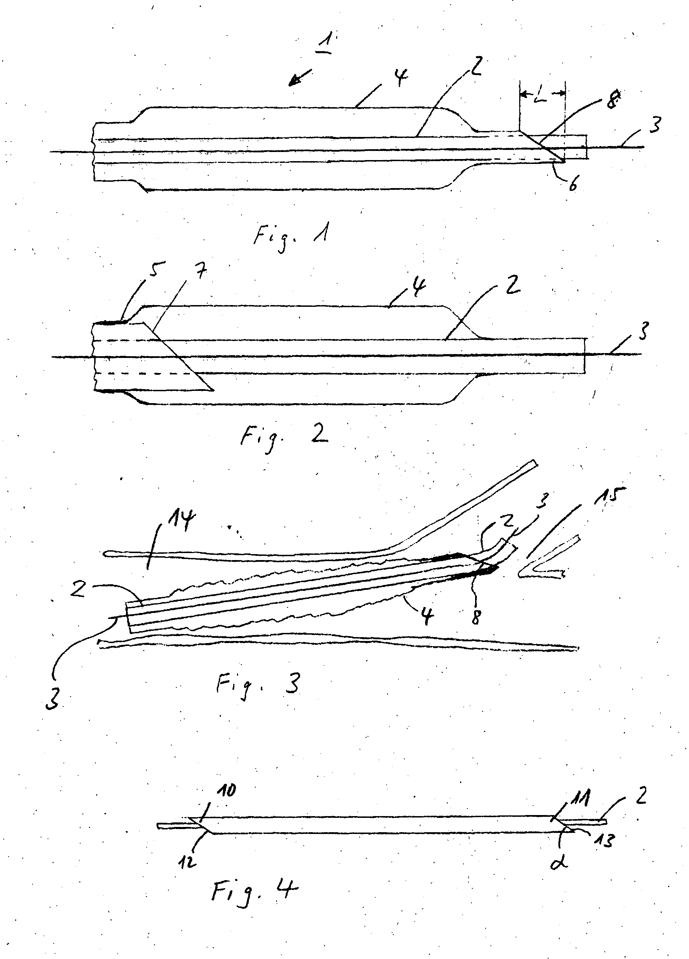

Dir Figur 1 zeigt die erfindungsgemässe Katheteranordnung 1. Die Katheteranordnung 1 weist einen Innenschaft 2, in dem der Führungsdraht 3 angeordnet ist, auf. Der Führungsdraht 3 und der Innenschaft 2 bilden gemeinsam eine Führungseinrichtung für die Katheteranordnung 1.

Die Katheteranordnung 1 weist weiterhin einen Ballon 4 auf. Am distalen Ende 6 des Ballons 4 ist ein schräger Bereich 8 angeordnet, wobei der schräge Bereich 8 des Ballons 4 mit dem Innenschaft 2 verschweisst oder verklebt ist.

Am proximalen Ende 5 des Ballons 4 kann ebenfalls ein schräger Bereich 7 angeordnet sein. Das proximale Ende 5 des Ballons 4 ist ebenfalls mit dem Innenschaft 2, wie in Figur 2 gezeigt, verschweisst. An den Ballonenden 5 und 6 können jeweils ein oder zwei schräge Bereiche 7, 8 angeordnet sein.

Die Längen L der schrägen bereiche bewegen sich im Bereich 0,5 bis 50mm, vorzugsweise 2 bis 6 mm.

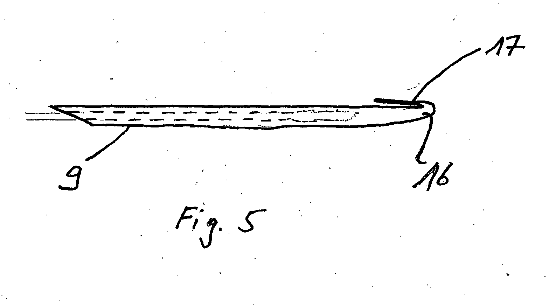

In Figur 3 ist die Funktionsweise der erfindungsgemässen Katheternordung 1 dargestellt. Die Figur 3 zeigt in vereinfachter Weise beispielsweise eine Arterie 14 mit einer Verzweigung 15. Die Katheteranordnung 1 soll von der Arterie 14 in diese Verzweigung 15 geführt werden. Hierbei wird zunächst der Führungsdraht 3 in die Abzweigung 15 vorgeschoben. Die Katheteranordnung 1 wird nunmehr über den Führungsdraht 3 transportiert. Aufgrund des schrägen Bereichs 8 wird wegen der eingestellten Steifigkeit verhindert, dass der Führungsdraht 3 aus der Verzweigung 15 herausgedrückt wird. Denn die Steifigkeit bei den bekannten Katheteranordnungen mit geradem Ende ist grösser als die Steifigkeit mit der erfindungsgemässen Schräge 8. Der in Figur 3 dargestellte Ballon 4 ist noch zusammengefaltet und wird erst an der zu behandelten Stelle mit einem Medium zum Ausdehnen beaufschlagt.

Die Figur 4 zeigt die Katheteranordnung 1 mit einer Schutzhülle 9. Die Schutzhülle 9 weist an jedem Ende 10, 11 schräge Bereiche 12 und 13 auf. Diese schrägen Bereiche 12, 13 dienen dazu einen optimal gefalteten Ballon 4 zu ermöglichen, indem die Schutzhülle 9 mittels einer Drehbewegung auf die Katheteranordnung aufgebracht wird. Der Winkel α der schrägen Bereiche 12 und 13 beträgt 30° bis 40°.

In Figur 5 ist eine Schutzhülle 9 gezeigt, die einen nicht sichtbaren Ballon verdeckt, wobei die Schutzhülle so auf den Ballon aufgebracht wird das ein Ende 16 das nicht sichtbare Ballonende 11 überragt. Dieses Ende 16 der Schutzhülle 9 wird in Richtung Ballon abgeknickt. Diese Abknickung 17 soll so ausgeführt sein, dass sie gut sichtbar ist, um eine nicht gewollte Einführung des Ballons mit der Schutzhülle zu vermeiden.

### Bezugszeichenliste

- 1: Katheteranordnung
- 2: Innenschaft
- 3: Führungsdraht
- 4: Ballon
- 5: proximales Ende
- 6: distales Ende
- 7: schräger Bereich
- 8: schräger Bereich
- 9: Schutzhülle
- 10: Ende
- 11: Ende
- 12: schräger Bereich
- 13: schräger Bereich
- 14: Arterie
- 15: Verzweigung
- 16: Ende
- 17: Abknickung

- L: Länge
- α: Winkel

## Patentansprüche

1. Katheteranordnung (1) mit einem Innenschaft (2) zur Aufnahme eines Führungsdrahtes (3) und eines expandierbaren Ballons (4), **dadurch gekennzeichnet, dass** der Ballon (4) am proximalen und/oder distalen Ende (5, 6) einen schrägen Bereich (7, 8) aufweist.

2. Katheteranordnung nach Anspruch 1, **dadurch gekennzeichnet, dass** der schräge Bereich (7, 8) eine Länge L von 0,5 bis 50 mm, vorzugsweise 2 bis 6 mm, aufweist.

3. Katheteranordnung nach Anspruch 1 oder 2, **dadurch gekennzeichnet, dass** der Innenschaft (2) am proximalen Ende (5) mit dem Ballon (4) verschweisst oder verklebt ist.

4. Katheteranordnung nach Anspruch 1 oder 2, **dadurch gekennzeichnet, dass** der Innenschaft (2) am distalen Ende (6) mit dem Ballon (4) verschweisst oder verklebt ist.

5. Katheteranordnung nach mindestens einem der vorherigen Ansprüche, **dadurch gekennzeichnet, dass** die Katheteranordnung (1) eine Schutzhülle (9) aufweist, wobei die Schutzhülle (9) über den geeignet gefalteten Ballon (4) angeordnet ist.

6. Katheteranordnung nach Anspruch 5, **dadurch gekennzeichnet, dass** die Schutzhülle (9) an einem und/oder beiden Enden (10, 11) jeweils einen schrägen Bereich (12, 13) aufweist.

7. Katheteranordnung nach Anspruch 5 oder 6, **dadurch gekennzeichnet, dass** der schräge Bereich (12, 13) einen Winkel α von 30° bis 40° aufweist.

8. Katheteranordnung nach mindestens einem der Ansprüche 5, 6 oder 7, **dadurch gekennzeichnet, dass** die Schutzhülle (9) durch Drehen auf den Ballon (4) aufbringbar ist, wobei durch das Drehen der Schutzhülle (9) auf den Ballon (4) dieser geeignet gefaltet wird.

9. Katheteranordnung nach mindestens einem der Ansprüche 5 bis 8, **dadurch gekennzeichnet, dass** die Schutzhülle (9) an dem über den Ballon (4) überstehenden Ende (16) eine Abknickung (17) aufweist.
